# EUROPÄISCHE PATENTSCHRIFT

(11) **EP 4 279 034 B1**
(45) Veröffentlichungstag und Bekanntmachung des Hinweises auf die Patenterteilung: **20.05.2026**
(21) Anmeldenummer: 23202257.4
(22) Anmeldetag: 18.12.2020
(51) Int. Cl.: A61F 2/60, A61F 2/68, A61F 5/01, A61F 2/50

(54) **GELENK FÜR EINE ORTHOPÄDIETECHNISCHE EINRICHTUNG**
JOINT FOR AN ORTHOPEDIC DEVICE
ARTICULATION POUR UN DISPOSITIF ORTHOPÉDIQUE

(30) Priorität: 20.12.2019 DE 102019135544
(43) Veröffentlichungstag der Anmeldung: 22.11.2023
(62) Teilanmeldung aus: 20842567.8
(73) Patentinhaber: Ottobock SE & Co. KGaA, 37115 Duderstadt (DE)
(72) Erfinder: LÜRSSEN, Marcus, 37115 Duderstadt (DE); BRAND, David, 37115 Duderstadt (DE); TÜTTEMANN, Markus, 37115 Duderstadt (DE); MÜLLER, André, 37115 Duderstadt (DE); RÖHRICHT, Marie-Luise, 37115 Duderstadt (DE)
(74) Vertreter: Gramm, Lins & Partner Patent- und Rechtsanwälte PartGmbB

(56) Entgegenhaltungen:
- GB-A- 120 445
- US-A- 2 442 151
- US-A- 5 653 680
- US-A1- 2019 008 672

## Beschreibung

Die Erfindung betrifft ein Gelenk für eine orthopädietechnische Einrichtung, wobei das Gelenk ein erstes Element, einen Federträger mit wenigstens einem Federelement, der an dem ersten Element montiert ist, und ein zweites Element aufweist.

Ein derartiges Gelenk ist beispielsweise in Form eines Knöchelgelenkes aus der DE 10 2010 014 334 A1 und der DE 10 2015 112 283 A1 bekannt. Derartige Knöchelgelenke können bei Bein- oder Unterschenkelorthesen verwendet werden. Dabei kann es aus therapeutischen Gründen sinnvoll sein, die Länge der Schwenkbewegung, also den maximal möglichen Schwenkwinkel des zweiten Elementes relativ zum ersten Element, zu begrenzen und beispielsweise in einer oder beiden entgegengesetzten Schwenkrichtungen jeweils einen Anschlag vorzusehen. Um ein zu hartes Anschlagen an diesen Anschlägen zu vermeiden, sind die Anschläge in der Regel federbelastet und damit gedämpft ausgeführt. Diese Federung sorgt zudem dafür, dass ein Verschwenken des Gelenkes für die orthopädietechnische Einrichtung nur möglich ist, wenn die durch das wenigstens eine Federelement aufgebrachte Kraft überwunden wird. Auch dies kann zu Rehabilitations- und Trainingszwecken sinnvoll sein.

Die Stärke der durch das wenigstens eine Federelement aufgebrachten Kraft ist dabei oftmals und vorzugsweise mittels einer sogenannten Vorspannung einstellbar ausgebildet. Aus der DE 10 2017 112 997 ist ein entsprechendes Gelenk bekannt, bei dem sowohl die Vorspannung als auch ein Eingriffswinkel im montierten Zustand des Gelenkes einstellbar ist. Unter dem Eingriffswinkel wird der Winkel zwischen dem ersten Element und dem zweiten Element verstanden, ab dem eine weitere Verschwenkung in eine jeweilige Schwenkrichtung nur gegen die vom Federelement aufgebrachte Kraft möglich ist. Zuvor kann ein sogenannter Freilaufbereich angeordnet sein, bei dem das erste Element relativ zum zweiten Element verschwenkt werden kann, ohne dass eine von dem wenigstens einen Federelement aufgebrachte Kraft überwunden werden muss.

Aus dem Stand der Technik bekannte Gelenke verfügen über zwei Federträger, die jeweils über wenigstens ein Federelement verfügen, wobei an dem jeweiligen Federträger ein Kraftübertragungselement angeordnet ist, das mit dem zweiten Element in Kontakt kommt. Beim weiteren Verschwenken des zweiten Elementes relativ zum ersten Element in die jeweilige Schwenkrichtung wird das Kraftübertragungselement bewegt und führt so beispielsweise zu einer Kompression eines Tellerfederstapels oder einer Schraubenfeder. Nachteilig ist jedoch, dass diese Art von Gelenk einen relativ großen Bauraum benötigt, was insbesondere bei Knöchelelementen von Nachteil ist, da sie gegebenenfalls innerhalb eines Schuhs getragen werden. Zudem bedeutet die Verwendung zweier Federträger mit jeweils wenigstens einem Federelement auch ein hohes Gewicht der Konstruktion, was ebenfalls nachteilig ist.

US 5 653 680 A zeigt eine Handgelenkstütze mit einem Unterarmteil und einem Handteil, die über mehrere Federdämpfer mit dem Unterteil verbunden ist. An einem Federdämpfer ist ein elastisches Zugelement angeordnet, um zusätzliche Kräfte und Widerstände aufzubringen.

US 2 442 151 A zeigt eine Knöchelkonstruktion mit einem Beinelement und einem Fußelement, die über ein Kugelgelenk miteinander verbunden sind. Posterior des Kugelgelenks ist eine Federeinheit mit zwei Federn zwischen dem Beinelement und dem Fußelement angeordnet, mit der unter anderem die Fersenposition eingestellt werden kann.

Der Erfindung liegt daher die Aufgabe zugrunde, ein Gelenk gemäß dem Oberbegriff des Anspruchs 1 so weiter zu entwickeln, dass es klein und leicht ausgeführt werden kann.

Die Erfindung löst die gestellte Aufgabe durch ein Gelenk für eine orthopädietechnische Einrichtung gemäß dem Oberbegriff des Anspruchs 1, das sich dadurch auszeichnet, dass das zweite Element in eine erste Schwenkrichtung gegen eine von dem wenigstens einen Federelement aufgebrachte erste Kraft und in eine entgegengesetzte zweite Schwenkrichtung gegen eine von dem wenigstens einen Federelement aufgebrachte zweite Kraft schwenkbar an dem ersten Element gelagert ist. Durch das wenigstens eine Federelement, das an einem einzigen Federträger angeordnet ist, werden folglich in beiden Schwenkrichtungen die jeweils verwendeten Kräfte aufgebracht. Dadurch kann ein gesamter Federträger eingespart und auf diese Weise die Konstruktion mit relativ kleinem benötigten Bauraum und wenig Eigengewicht ausgebildet werden. Das erste Element und das zweite Element sind um eine Schwenkachse schwenkbar aneinander angeordnet. Das Gelenk weist eine hydraulische Dämpfereinheit auf, durch die eine Verschwenkung des ersten Elementes relativ zu dem zweiten Element in zumindest eine Schwenkrichtung, vorzugsweise in beiden Schwenkrichtungen gedämpft wird, wobei der Federträger auf einer Seite des Gelenks und die hydraulische Dämpfereinheit auf der anderen Seite des Gelenks angeordnet ist.

Vorteilhafterweise verfügt der Federträger über wenigstens zwei Federelemente, von denen eines die erste Kraft und eines die zweite Kraft aufbringt. Dabei kann es sich beispielsweise um Druckfederelemente handeln. Wird also das zweite Element relativ zum ersten Element in die erste Schwenkrichtung verschwenkt, wird in diesem Fall beispielsweise eines der Federelemente komprimiert, was nur gegen die wirkende Federkraft nötig ist. Diese ist in diesem Fall die erste Kraft. Beim Verschwenken des zweiten Federelementes in die entgegengesetzte zweite Schwenkrichtung relativ zum ersten Element wird das zweite Federelement komprimiert, wodurch die zweite Kraft aufgebracht wird. Durch unterschiedliche Ausgestaltungen der beiden Federelemente, die vorteilhafterweise austauschbar, besonders vorteilhafterweise unabhängig voneinander austauschbar sind, lässt sich die erste Kraft und die zweite Kraft vorzugsweise unabhängig voneinander einstellen. Dies ist beispielsweise sinnvoll, wenn eine Bewegung des Gelenkes in eine der beiden Schwenkrichtungen stärker gedämpft sein soll als die Bewegung in die entgegengesetzte andere Schwenkrichtung.

In einer bevorzugten Ausgestaltung sind die beiden Federelemente ineinander oder hintereinander angeordnet. Die Anordnung ineinander ist in besonders einfacher Weise möglich, wenn die beiden Federelemente beispielsweise Schraubenfedern, Schraubentellerfedern oder Tellerfederstapel sind. Alle diese Federn verfügen in ihrem Innenraum über einen Hohlraum, in dem das jeweils andere Federelement angeordnet werden kann. Dadurch wird der benötigte Bauraum weiter reduziert. Alternativ oder zusätzlich dazu können jedoch selbstverständlich auch die beiden Federelemente nebeneinander oder in Kompressionsrichtung hintereinander angeordnet sein. Insbesondere Schraubenfedern, Schraubendruckfedern und Schraubentellerfedern weisen eine Längsachse auf. Diese ist vorzugsweise die Richtung, um die sich die schraubenförmige Wickelung des jeweiligen Schraubenelementes wickelt. Sie ist vorzugsweise identisch mit der Kompressions- oder Dehnungsrichtung des Federelementes. In dieser Richtung sind die wenigstens zwei Federelemente vorzugsweise hintereinander angeordnet. Dabei sind die wenigstens zwei Federelemente vorzugsweise so ausgerichtet, dass ihre beiden Längsrichtungen parallel zueinander verlaufen und besonders bevorzugt identisch, also koaxial sind.

In einer bevorzugten Ausgestaltung sind die wenigstens zwei Federelemente derart angeordnet und eingerichtet, dass eines der beiden Federelemente zum Aufbringen der jeweiligen Kraft in Zugrichtung belastet wird und bevorzugt ein Zugfederelement ist und eines der beiden Federelemente zum Aufbringen der jeweiligen Kraft in Druckrichtung belastet wird und vorzugsweise ein Druckfederelement ist. Besonders bevorzugt sind die beiden Federelemente Druckfedern. Dies sind insbesondere Federelemente, die durch Druck mit Energie aufgeladen, insbesondere in der räumlichen Ausdehnung komprimiert werden, und dann eine Druckkraft ausüben.

Wird das zweite Element in eine der beiden Schwenkrichtungen gegen die Kraft eines der wenigstens zwei Federelemente verschwenkt, wird dieses Federelement komprimiert, wenn es in Druckrichtung belastet wird und gedehnt, wenn es in Zugrichtung belastet wird.

In einer besonders bevorzugten Ausgestaltung ist nur ein einziges Federelement vorhanden, das sowohl Druckfederelement als auch Zugfederelement ist. Beim Verschwenken des zweiten Elementes in die erste Schwenkrichtung wird dabei das Federelement beispielsweise komprimiert, während es beim Verschwenken des zweiten Elementes in die entgegengesetzte zweite Schwenkrichtung expandiert, also verlängert wird. Durch geeignete Wahl der jeweiligen Federkennlinie lassen sich auch hier unterschiedliche erste und zweite Kräfte einstellen. Alternativ dazu kann auch ein einziges Federelement verwendet werden, das sowohl beim Verschwenken des zweiten Elementes in die erste Richtung als auch beim Verschwenken des zweiten Elementes in die zweite Richtung druckbelastet, also vorzugsweise komprimiert, wird. In diesem Fall ist allerdings für beide Bewegungsrichtungen nur eine Vorspannung, eine Federstärke und damit ein Kraftverlauf verwendbar.

Bevorzugt kann eine Vorspannung wenigstens eines der Federelemente, bevorzugt aller Federelemente, eingestellt werden. Dies ist besonders bevorzugt unabhängig voneinander möglich. Dazu ist vorzugsweise wenigstens ein Einstellelement, besonders bevorzugt wenigstens ein Einstellelement pro Federelement vorhanden. **In** einer besonders bevorzugten Ausgestaltung ist die jeweilige Vorspannung auch im montierten Zustand des Gelenks einstellbar. Dazu ist es nötig, dass das jeweilige Einstellelement von außen zugänglich ist, auch wenn das Gelenk montiert ist. Dies hat den großen Vorteil, dass zur individuellen Einstellung der Dämpfungen das Gelenk und damit die orthopädietechnische Einrichtung, an der das Gelenk angeordnet ist, nicht demontiert werden muss, so dass die Einstellung von beispielsweise einem Orthopädiemechaniker oder auch vom Patienten selbst auf besonders einfache Weise vorgenommen werden kann.

In einer bevorzugten Ausgestaltung ist das zweite Element mit einem Kraftübertragungselement des Federträgers so verbunden, dass Zugkräfte und Druckkräfte übertragbar sind. Wird das zweite Element relativ zum ersten Element in die erste Schwenkrichtung verschwenkt, werden beispielsweise Druckkräfte vom zweiten Element auf das Kraftübertragungselement übertragen. Das Kraftübertragungselement wird mit dem wenigstens einen Federelement gekoppelt, und komprimiert oder expandiert das Federelement, wodurch die erste Kraft ausgeübt wird. Dabei wird das Federelement komprimiert, wenn es in Druckrichtung belastet wird und gedehnt, wenn es in Zugrichtung belastet wird. Beim Verschwenken des zweiten Bauteils in die entgegengesetzte zweite Schwenkrichtung werden Zugkräfte vom zweiten Element auf das Kraftübertragungselement des Federträgers übertragen. Auch dies hat eine Bewegung des Kraftübertragungselementes relativ zum Rest des Federträgers und insbesondere zum Federelement zur Folge, wodurch das Federelement komprimiert oder expandiert wird, wodurch die zweite Kraft aufgebracht wird. Ist nur ein Federelement vorhanden, wird dieses beispielsweise beim Verschwenken des zweiten Elementes in die erste Schwenkrichtung komprimiert, also in Druckrichtung belastet, und beim Verschwenken des zweiten Elementes in die zweite Schwenkrichtung gedehnt, also in Zugrichtung belastet. Selbstverständlich ist auch die umgekehrte Ausgestaltung möglich. Selbstverständlich ist es auch möglich, ein einzelnes Federelement so zu verwenden, dass es unabhängig von der Schwenkrichtung des zweiten Elementes gedehnt, also in Zugrichtung belastet, oder komprimiert, also in Druckrichtung belastet wird.

Alternativ dazu ist es selbstverständlich auch möglich, dass das zweite Element mit zwei Kraftübertragungselementen des Federträgers verbunden ist, die beispielsweise beide Druckkräfte oder beide Zugkräfte übertragen. Dabei wird beim Verschwenken des zweiten Elementes in die erste Schwenkrichtung eine Druck- oder Zugkraft über das erste Kraftübertragungselement übertragen, während beim Verschwenken des zweiten Elementes in die entgegengesetzte zweite Schwenkrichtung die jeweilige Kraft über das zweite Kraftübertragungselement übertragen wird.

Eine solche Verbindung zwischen dem zweiten Element und dem Kraftübertragungselement des Federträgers stellt eine eigene Erfindung dar, die insbesondere auch separat in einem Gelenk gemäß dem Oberbegriff des Anspruchs 1 verwendbar ist. Es ist nicht notwendig, dass das zweite Element in eine erste Schwenkrichtung gegen eine erste Kraft und in die entgegengesetzte zweite Schwenkrichtung gegen eine zweite Kraft schwenkbar ist und/oder, dass diese beiden Kräfte von einem oder mehreren Federelementen des gleichen Federträgers ausgeübt werden. Vielmehr kann eine solche Verbindung auch sinnvoll verwendet werden, wenn das Gelenk für eine orthopädietechnische Einrichtung ein erstes Element, einen Federträger mit wenigstens einem Federelement, der an dem ersten Element montiert ist, und ein zweites Element aufweist. Auch bei einem derartigen Gelenk ist es von Vorteil, wenn durch die Verbindung zwischen dem zweiten Element und einem Kraftübertragungselement des Federelementes Zugkräfte und Druckkräfte übertragen werden können.

Vorteilhafterweise weist das wenigstens eine Federelement eine Schraubenfeder, eine Schraubentellerfeder, einen Tellerfederstapel und/oder ein gummielastisches Element, insbesondere einen Elastomerblock auf. Sind mehr als ein Federelement vorhanden, können unterschiedliche Arten von Federelementen miteinander kombiniert oder gleiche Federelemente verwendet werden.

Bevorzugt bringt das wenigstens eine Federelement die erste Kraft erst ab einem ersten Eingriffswinkel und/oder die zweite Kraft erst ab einem zweiten Eingriffswinkel auf das zweite Element auf. Dies bedeutet beispielsweise bei der ersten Kraft in die erste Schwenkrichtung, dass beim Verschwenken des zweiten Elementes in die erste Schwenkrichtung erst ab Erreichen des ersten Eingriffswinkels die erste Kraft überwunden werden muss. Zuvor ist das Verschwenken ohne eine wirkende Kraft möglich. Analog kann es von Vorteil sein, wenn die zweite Kraft beim Verschwenken des zweiten Elementes in die entgegengesetzte zweite Schwenkrichtung erst ab Erreichen eines zweiten Eingriffswinkels aufgebracht wird.

Das Gelenk verfügt über eine hydraulische Dämpfereinheit, durch die ein Verschwenken des ersten Elementes relativ zu dem zweiten Element in zumindest einer Schwenkrichtung, vorzugsweise in beide Schwenkrichtungen gedämpft wird. Eine solche hydraulische Dämpfereinheit umfasst vorzugsweise wenigstens einen Zylinder, in dem sich ein Kolben befindet, der entlang einer Längsrichtung des Zylinders verschoben werden kann. Vorzugsweise verfügt der Zylinder über zwei Zylinderkammern, die sich vorzugsweise auf zwei Seiten des Kolbens befinden und miteinander in fluidtechnischer Verbindung stehen. Wird nun der Kolben innerhalb des Zylinders bewegt, wird das Hydraulikmedium, das sich in der hydraulischen Dämpfereinheit befindet, von einer der Kammern in die zweite Kammer gepumpt. Dabei stellt die fluidtechnische Verbindung, beispielsweise ein Kanal oder ein Schlauch, einen Strömung Widerstand dar, durch den die Dämpfung hervorgerufen wird. Selbstverständlich kann die hydraulische Dämpfereinheit auch zwei Zylinder mit jeweils einer Kammer aufweisen.

Vorzugsweise ist die Dämpfung einstellbar. Besonders bevorzugt ist die Dämpfung für beide Schwenkrichtungen separat voneinander einstellbar. Eine einstellbare Dämpfung kann beispielsweise erreicht werden, indem in der fluidtechnischen Verbindung, also beispielsweise dem Kanal oder dem Schlauch, ein Drosselventil angeordnet ist, durch das der Strömungswiderstand, der durch die Fluidtechnische Verbindung hervorgerufen wird, einstellbar ist. Soll die Dämpfung für beide Schwenkrichtungen separat einstellbar sein, können zwei fluidtechnische Verbindungen zwischen den beiden Kammern vorhanden sein, die jeweils über ein Drosselventil verfügen. Durch Rückschlagventile, die in den beiden fluidtechnischen Verbindungen positioniert sind, wird erreicht, dass die jeweilige Fluidtechnische Verbindung nur in einer Richtung durchströmbar ist.

Anhand der beiliegenden Zeichnungen werden nachfolgend einige Ausführungsbeispiele der vorliegenden Erfindung näher erläutert. Es zeigen:
- Figuren 1 - 3 -: eine Schnittdarstellung durch ein Gelenk gemäß einem ersten Ausführungsbeispiel der vorliegenden Erfindung in unterschiedlichen Positionen,
- Figur 4 -: eine schematische Schnittdarstellung durch ein weiteres Gelenk,
- Figur 5 -: eine Schnittdarstellung durch ein Gelenk gemäß einem weiteren Ausführungsbeispiel,
- Figuren 6 bis 8 -: schematische Schnittdarstellungen durch Gelenke gemäß weiterer Ausführungsbeispiele der vorliegenden Erfindung,
- Figuren 9 und 10 -: Darstellung eines Gelenkes gemäß einem weiteren Ausführungsbeispiel der vorliegenden Erfindung und
- Figuren 11 bis 13-: Darstellungen eines Teiles des Gelenkes aus den Figuren 9 und 10 in verschiedenen Stellungen.

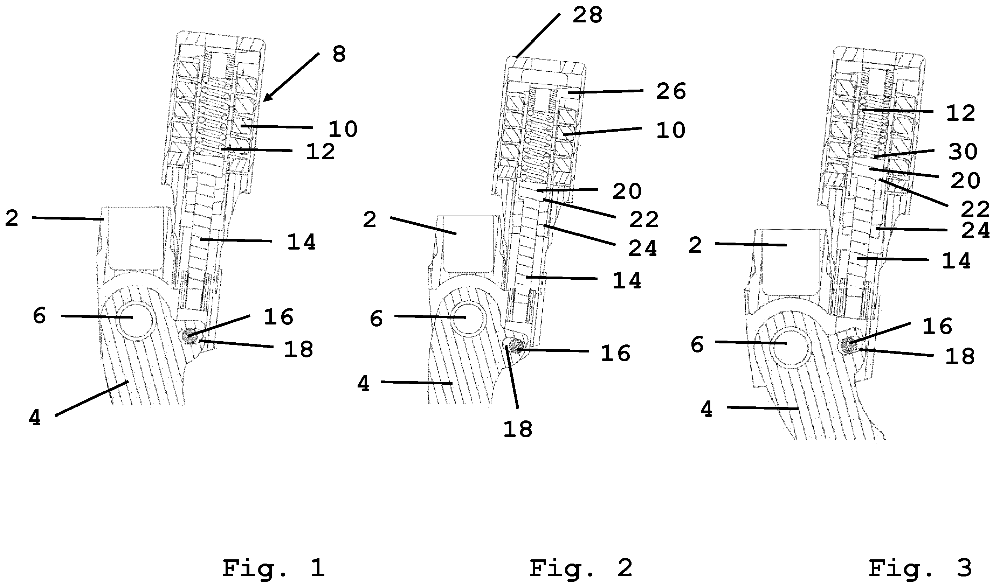

Figur 1 zeigt eine Schnittdarstellung durch ein Gelenk gemäß einem ersten Ausführungsbeispiel der vorliegenden Erfindung, das ein erstes Element 2 und ein zweites Element 4 aufweist, die um eine Schwenkachse 6 schwenkbar aneinander angeordnet sind. Am ersten Bauteil 2 befindet sich ein Federträger 8, der ein erstes Federelement 10 und ein zweites Federelement 12 aufweist. Der Federträger 8 verfügt zudem über ein Kraftübertragungselement 14, an dessen unteren Ende sich ein Pin 16 befindet, der in ein dafür vorgesehenes Langloch 18 am zweiten Element 4 eingreift und so eine Verbindung zwischen dem Kraftübertragungselement 14 und dem zweiten Element 4 herstellt, durch die sowohl Druckkräfte als auch Zugkräfte übertragen werden können.

Die Figuren 2 und 3 zeigen die Darstellung aus Figur 1, wobei das Gelenk nun in unterschiedliche Positionen bewegt wurde.

Figur 2 zeigt, dass das zweite Element 4 relativ zum ersten Element 2 um die Schwenkachse 6 im Uhrzeigersinn verschwenkt wurde. Dadurch wird über den Pin 16 im Langloch 18 eine Zugkraft auf das Kraftübertragungselement 14 ausgeübt, dass sich daher nach unten bewegt. Es verfügt über einen Kopf 20, der über einen ringförmigen Anschlag 22 verfügt. Der ringförmige Anschlag 22 ist sowohl in Figur 1 als auch in Figur 2 in Kontakt mit einem Kompressionsbauteil 24, das sich im Innern des ersten Federelementes 10 erstreckt und an dessen in Figur 2 oberen Ende ein Kompressionskopf 26 angeordnet ist. Bewegt sich das Kraftübertragungselement 14 durch die Schwenkbewegung des zweiten Elementes 4 relativ zum ersten Element 2 nach unten, bewegt sich auch das Kompressionsbauteil 24 und der sich daran befindende Kompressionskopf nach unten, wodurch das erste Federelement 10 komprimiert und die erste Kraft aufgebracht wird. Sowohl das Kraftübertragungselement 14 als auch das Kompressionsbauteil mit dem Kompressionskopf können jeweils einstückig oder mehrstückig in Form mehrerer miteinander verbundener Bauteile ausgebildet sein.

Im Vergleich zur Situation in Figur 1 ist in Figur 2 deutlich zu erkennen, dass der Kompressionskopf 26 eine Bewegung nach unten ausgeführt hat und nun einen Zwischenraum bildet zwischen dem Kompressionskopf 26 und dem oberen Ende einer Hülse 28, die eine äußere Begrenzung des Federträgers darstellt.

Figur 3 zeigt die umgekehrte Situation. Das zweite Element 4 ist relativ zum ersten Element 2 um die Schwenkachse 6 herum gegen den Uhrzeigersinn verschwenkt worden. Dadurch wird über den Pin 16 im Langloch 18 eine Druckkraft auf das Kraftübertragungselement 14 ausgeübt, das nun nach oben verschoben wird. Man erkennt, dass der Anschlag 22 des Kopfes 20 des Kraftübertragungselementes 14 nicht mehr an dem entsprechenden Kompressionsbauteil 24 anliegt. Stattdessen hat eine Kontaktfläche 30 des Kopfes 20 das zweite Federelement 12 nach oben verschoben und dabei komprimiert, wobei die zweite Kraft aufgebracht wurde.

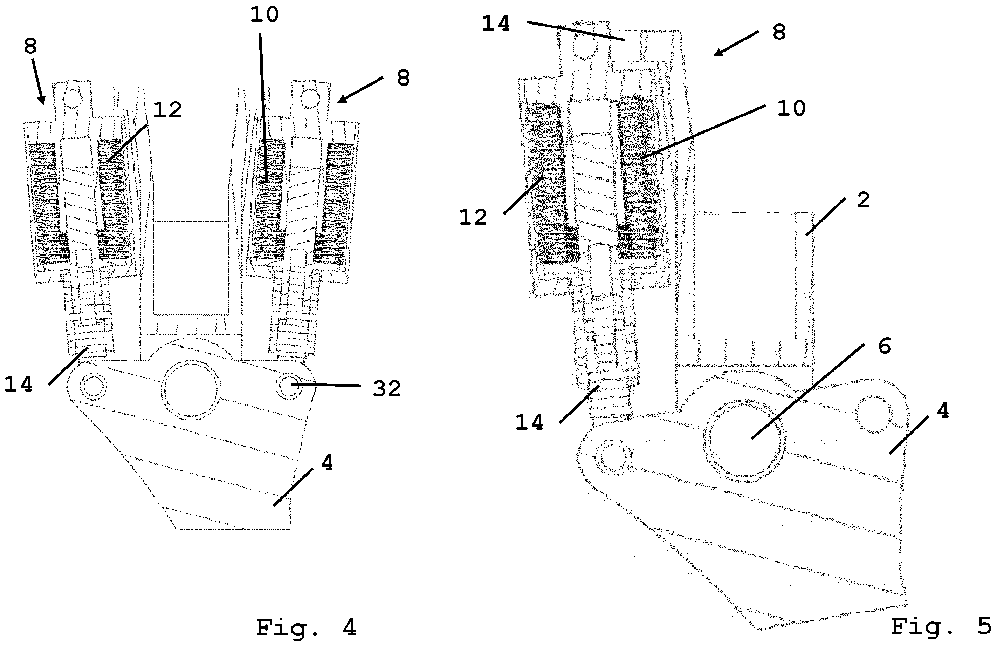

Figur 4 zeigt eine Ausgestaltung des Gelenkes, bei der zwei Federträger 8 am zweiten Element 4 angeordnet sind. Sie tragen jeweils eines der Federelement 10, 12, die im gezeigten Ausführungsbeispiel beide als Druckfedern ausgebildet sind. Da die Kraftübertragungselemente 14 so am zweiten Element 4 angeordnet sind, dass Druckkräfte und Zugkräfte übertragen werden können, ist diese Ausführungsform ausreichend. Alternativ dazu könnten auch beide Federelemente 10, 12 als Zugfeder ausgebildet werden. Selbstverständlich können auch Federträger 8 verwendet werden, die ein erstes Federelement 10 und ein zweites Federelement 12 aufweisen, die wiederum jeweils als Zugelemente, jeweils als Druckelemente oder als verschiedene Elemente, also ein Zugelement und ein Druckelement, ausgebildet sein können.

Die Ösen 32 werden mit Bohrungen im zweiten Element 4 in Überdeckung gebracht. Anschließend wird ein Stift oder Bolzen durch diese Öffnungen hindurchgeschoben, sodass die in Figur 4 und 5 gezeigte Verbindung erreicht wird.

Figur 5 zeigt für eine andere Ausgestaltung des Gelenks mit dem ersten Bauteil 2, dem zweiten Bauteil 4 und der Schwenkachse 6, wie eine Kopplung des Kraftübertragungselementes 14 an das zweite Element 4 erfolgen kann. Das Kraftübertragungselement 14 ist an dem zweiten Element 4 gelenkig angeordnet und wird somit sowohl bei einem Verschwenken des zweiten Elementes 4 relativ zu dem ersten Element 2 im Uhrzeigersinn und gegen den Uhrzeigersinn bewegt.

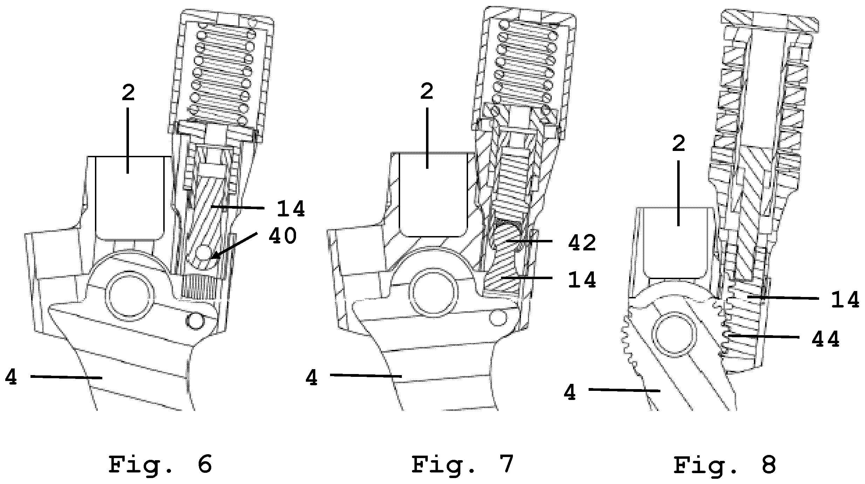

Die Figuren 6, 7 und 8 zeigen jeweils verschiedene Ausführungsformen eines Federträgers, der in ein Gelenk mit dem ersten Bauteil 2 und dem zweiten Bauteil 4 montiert ist. Dabei liegt der Fokus der Darstellung insbesondere auf der Befestigung des jeweiligen Kraftübertragungselementes 14 am zweiten Element 4. Während in Figur 6 ein Pleuelgelenk 40 verwendet wird verfügt die Ausgestaltung in Figur 7 über ein Kugelgelenk 42 und Figur 8 über eine entsprechende Verzahnung 44.

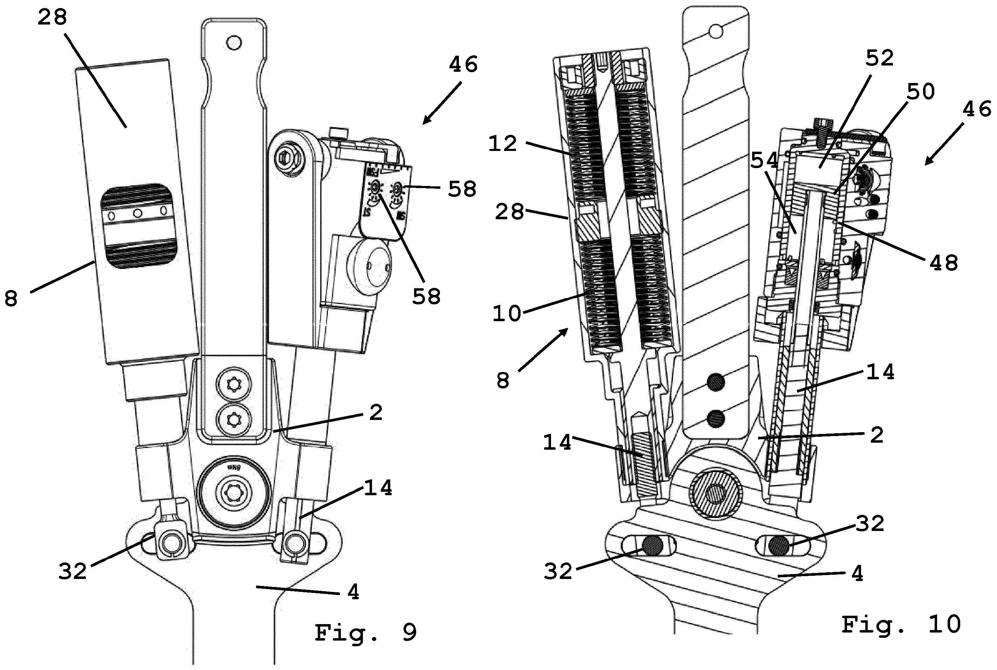

Figur 9 zeigt eine schematische Darstellung eines Gelenkes mit dem ersten Element 2 und dem zweiten Element 4. Im linken Bereich des Gelenkes, dass in Figur 9 in einer schematischen 3-D-Ansicht gezeigt ist, befindet sich der Federträger 8, bei dem die Öse 32, die mittels eines Pins oder Stiftes an einem Langloch des zweiten Elementes 4 angeordnet ist, dargestellt ist. Zudem ist die Hülse 28 gezeigt, die die Federelemente 10, 12 und die anderen Elemente und Bauteile enthalten kann. Auf der rechten Seite des Gelenkes ist eine hydraulische Dämpfereinheit 46 dargestellt.

Figur 10 zeigt das Gelenk aus Figur 9 in einer schematischen Schnittdarstellung. In der Hülse 28 sind das erste Federelement 10 sowie das zweite Federelement 12 dargestellt. Die hydraulische Dämpfereinheit 46 verfügt über einen Zylinder 48, in dem sich ein Kolben 50 bewegen kann, der den Innenraum des Zylinders 48 in eine erste Zylinderkammer 52 und eine zweite Zylinderkammer 54 teilt. Am Kolben 50 befindet sich eine Kolbenstange 56, die mit einem Kraftübertragungselement 14 verbunden ist. Dieses verfügt wie das Kraftübertragungselement 14 auf der linken Seite des Gelenkes, dass Teil des Federträgers 8 ist, über eine Öse 32, die über einen Pin oder Stift an einem zweiten Langloch des zweiten Elementes 4 befestigt ist.

Wird das zweite Element 4 relativ zum ersten Element 2 verschwenkt, wird auch das Kraftübertragungselement 14 bewegt, wodurch der Kolben 50 innerhalb des Zylinders 48 verschoben wird. Dadurch wird ein Hydraulikmedium von der ersten Zylinderkammer 52 in die zweite Zylinderkammer 54 oder umgekehrt gepumpt. Dazu sind zwischen den beiden Zylinderkammern 52, 54 Fluidverbindungen vorhanden, die aus Gründen der Übersichtlichkeit nicht dargestellt sind. Man erkennt in Figur 9 jedoch Einstelleinrichtungen 58, durch die nicht dargestellt Drosselventile geöffnet oder geschlossen werden können, sodass ein Strömungswiderstand, der durch die Drosselventile in den Fluidverbindungen erzeugt wird, einstellbar ist. Dadurch sind die Dämpfungen für Bewegungen der beiden Elemente 2, 4 relativ zueinander in beide Schwenkrichtungen separat voneinander einstellbar.

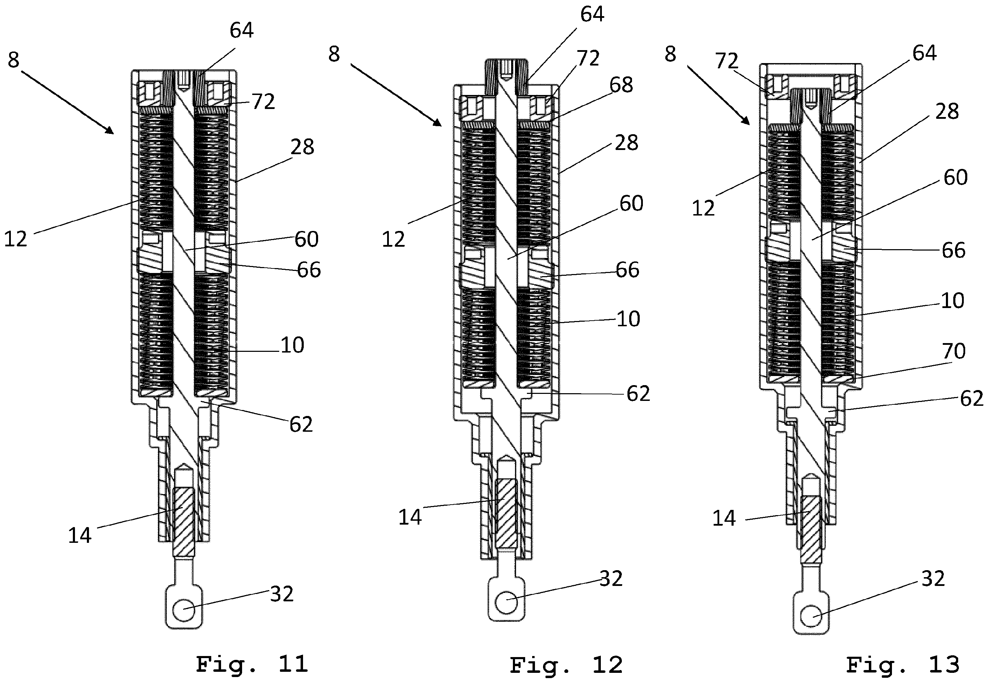

Die Figuren 11 bis 13 zeigen jeweils eine Schnittdarstellung durch den Federträger 8 mit der Hülse 28, wie er in dem Gelenk gemäß Figuren 9 und 10 verwendet wird. Im zentralen Bereich befindet sich das Kraftübertragungselement 14 an dessen unterem Ende sich die Öse 32 befindet. Das Kraftübertragungselement 14 ist an einer Mittelstange 60 befestigt, die über einen unteren Kompressionsvorsprung 62 und einen oberen Kompressionsvorsprung 64 verfügt. Im mittleren Bereich der Hülse 28 ist ein Anschlag 66 befestigt, der vorzugsweise stufenlos einstellbar ist. Zwischen dem Anschlag 66 und dem unteren Kompressionsvorsprung 62 erstreckt sich das erste Federelement 10 und zwischen dem Anschlag 66 und dem oberen Kompressionsvorsprung 64 ist das zweite Federelement 12 angeordnet.

Figur 11 zeigt den Federträger 8 in neutraler Stellung. Im Gegensatz dazu ist in Figur 12 das Kraftübertragungselement 14 gemeinsam mit der Mittelstange 60 nach oben verschoben worden, es wird also durch das zweite Element 4 ein Druck ausgeübt. Dadurch wird die Mittelstange sowie der an ihr befestigte obere Kompressionsvorsprung 64 und der untere Kompressionsvorsprung 62 nach oben bewegt. Das erste Federelement 10 wird daher zwischen dem unteren Kompressionsvorsprung 62 und dem Anschlag 66 komprimiert, so dass eine Rückstellkraft ausgeübt wird. Das zweite Federelement 12 hingegen bleibt unverändert und wird nicht vorgespannt, da sich der Abstand zwischen dem Anschlag 66 und einer oberen Anlagefläche 68, an der das zweite Federelement 12 anliegt, nicht ändert. Es wird also lediglich das erste Federelement 10 vorgespannt.

Figur 13 zeigt die umgekehrte Situation, in der auf die Öse 32 und damit auf das Kraftübertragungselement 14 und die Mittelstange 60 eine Zugkraft nach unten ausgeübt wird. Nun wird das zweite Federelement 12 komprimiert, da sich der Abstand zwischen dem Anschlag 66 und dem oberen Kompressionsvorsprung 64 verringert. Das erste Federelement 10 hingegen bleibt unverändert und wird nicht vorgespannt, da der Abstand zwischen dem Anschlag 66 und einer unteren Anlagefläche 70 unverändert bleibt.

Das Federelement 10 kann durch Verschieben des Anschlages 66 vorgespannt werden. Der Federelement 12 kann durch Verschieben eines oberen Anschlags 72 vorgespannt werden. In Abhängigkeit der Vorspannung des Federelementes 12 und dem Absinken des oberen Anschlags 72 muss gegebenenfalls der obere Kompressionsvorsprung 64 nachgestellt werden, so dass er auf der Anlagefläche 68 liegt. Die Statik und der Ausgangswinkel des Knöchelgelenksystems können über die Mittelstange 60 und das Kraftübertragungselement 14 aufgrund der Verbindung mittels Gewinde stufenlos eingestellt werden.

### Bezugszeichenliste

- 2: erstes Element
- 4: zweites Element
- 6: Schwenkachse
- 8: Federträger
- 10: erstes Federelement
- 12: zweites Federelement
- 14: Kraftübertragungselement
- 16: Pin
- 18: Langloch
- 20: Kopf
- 22: Anschlag
- 24: Kompressionsbauteil
- 26: Kompressionskopf
- 28: Hülse
- 30: Kontaktfläche
- 32: Öse
- 34: Kompressionsvorsprung
- 36: Formschlusselement
- 38: Öffnung
- 40: Pleuelgelenk
- 42: Kugelgelenk
- 44: Verzahnung
- 46: hydraulische Dämpfereinheit
- 48: Zylinder
- 50: Kolben
- 52: erste Zylinderkammer
- 54: zweite Zylinderkammer
- 56: Kolbenstange
- 58: Einstelleinrichtung
- 60: Mittelstange
- 62: unterer Kompressionsvorsprung
- 64: oberer Kompressionsvorsprung
- 66: Anschlag
- 68: obere Anlagefläche
- 70: untere Anlagefläche
- 72: oberer Anschlag

## Patentansprüche

1. Gelenk für eine orthopädietechnische Einrichtung, wobei das Gelenk
- ein erstes Element (2),
- einen Federträger (8) mit wenigstens einem Federelement (10, 12), der an dem ersten Element (2) montiert ist, und
- ein zweites Element (4) aufweist,
wobei das zweite Element (4) in eine erste Schwenkrichtung gegen eine von dem wenigstens einen Federelement (10, 12) aufgebrachte erste Kraft und in eine entgegengesetzte zweite Schwenkrichtung gegen eine von dem wenigstens einen Federelement (10, 12) aufgebrachte zweite Kraft schwenkbar an dem ersten Element (2) gelagert ist, wobei das erste Element (2) und das zweite Element (4) um eine Schwenkachse (6) schwenkbar aneinander angeordnet sind,
**dadurch gekennzeichnet, dass** das Gelenk eine hydraulische Dämpfereinheit (46) aufweist, durch die eine Verschwenkung des ersten Elementes (2) relativ zu dem zweiten Element (4) in zumindest eine Schwenkrichtung, vorzugsweise in beide Schwenkrichtungen gedämpft wird, wobei der Federträger (8) auf einer Seite des Gelenks und die hydraulische Dämpfereinheit (46) auf der anderen Seite des Gelenks angeordnet ist.

2. Gelenk nach Anspruch 1, **dadurch gekennzeichnet, dass** das zweite Element (4) mit einem Kraftübertragungselement (14) der hydraulischen Dämpfereinheit (46) so verbunden ist, dass Zugkräfte und Druckkräfte übertragbar sind.

3. Gelenk nach einem der vorstehenden Ansprüche, **dadurch gekennzeichnet, dass** der Federträger wenigstens zwei Federelemente aufweist, von denen eines die erste Kraft und eines die zweite Kraft aufbringt.

4. Gelenk nach Anspruch 3, **dadurch gekennzeichnet, dass** die beiden Federelemente (10, 12) ineinander oder hintereinander angeordnet sind.

5. Gelenk nach Anspruch 3 oder 4, **dadurch gekennzeichnet, dass** die beiden Federelemente (10, 12) Druckfedern sind.

6. Gelenk nach Anspruch 3 oder 4, **dadurch gekennzeichnet, dass** die wenigstens zwei Federelemente (10, 12) derart angeordnet und eingerichtet sind, dass eines der beiden Federelemente (10, 12) zum Aufbringen der jeweiligen Kraft in Zugrichtung belastet wird und bevorzugt ein Zugfederelement ist und eines der beiden Federelemente zum Aufbringen der jeweiligen Kraft in Druckrichtung belastet wird und bevorzugt ein Druckfederelement ist.

7. Gelenk nach einem der vorstehenden Ansprüche, **dadurch gekennzeichnet, dass** eine Vorspannung wenigstens eines der Federelemente (10, 12), bevorzugt aller Federelemente, einstellbar, bevorzugt unabhängig voneinander einstellbar ist.

8. Gelenk nach Anspruch 7, **dadurch gekennzeichnet, dass** die jeweilige Vorspannung im montierten Zustand des Gelenks einstellbar ist.

9. Gelenk nach dem Oberbegriff des Anspruchs 1, insbesondere nach einem der vorstehenden Ansprüche, **dadurch gekennzeichnet, dass** das zweite Element (4) mit einem Kraftübertragungselement (14) des Federträgers (8) so verbunden ist, dass Zugkräfte und Druckkräfte übertragbar sind.

10. Gelenk nach einem der vorstehenden Ansprüche, **dadurch gekennzeichnet, dass** das wenigstens eine Federelement (10, 12) eine Schraubenfeder, eine Schraubendruckfeder, eine Schraubentellerfeder, einen Tellerfederstapel und/oder ein gummielastisches Element, insbesondere einen Elastomerblock aufweist.

11. Gelenk nach einem der vorstehenden Ansprüche, **dadurch gekennzeichnet, dass** das wenigstens eine Federelement (10, 12) die erste Kraft erst ab einem ersten Eingriffswinkel und/oder die zweite Kraft erst ab einem zweiten Eingriffswinkel auf das zweite Element (4) aufbringt.

12. Gelenk nach einem der vorstehenden Ansprüche, **dadurch gekennzeichnet, dass** die Dämpfung einstellbar, vorzugsweise für beide Schwenkrichtungen separat voneinander einstellbar ist.

## Claims

1. A joint for an orthopedic device, wherein the joint comprises
- a first element (2),
- a spring support (8) with at least one spring element (10, 12) mounted on the first element (2), and
- a second element (4),
wherein the second element (4) is mounted to the first element (2) such that it is pivotable in a first pivot direction counter to a first force applied by the at least one spring element (10, 12) and in an opposite second pivot direction counter to a second force applied by the at least one spring element (10, 12), wherein the first element (2) and the second element (4) are arranged such that they can be pivoted about a pivot axis (6), **characterized in that** the joint comprises a hydraulic damping unit (46), by way of which a pivoting of the first element (2) relative to the second element (4) is damped in at least one pivot direction preferably in both pivot directions, wherein the spring support (8) is positioned on one side of the joint and the hydraulic damping unit (46) on the other side.

2. The joint according to claim 1, **characterized in that** the second element (4) is connected to a force transmission element (14) of the hydraulic damping unit (46) in such a way that tensile forces and compressive forces can be transmitted.

3. The joint according any of the preceding claims, **characterized in that** the spring support (8) has at least two spring elements (10, 12), one of which applies the first force and one of which applies the second force.

4. The joint according to claim 3, **characterized in that** the two spring elements (10, 12) are arranged one inside the other or one behind the other.

5. The joint according to claim 3 or 4, **characterized in that** the two spring elements (10, 12) are compression springs.

6. The joint according to claim 3 or 4, **characterized in that** the at least two spring elements (10, 12) are arranged and configured in such a way that one of the two spring elements (10, 12) is loaded in the tensile direction to apply the respective force and is preferably a tensile spring element, and one of the two spring elements is loaded in the compression direction to apply the respective force and is preferably a compression spring element.

7. The joint according to one of the preceding claims, **characterized in that** the respective pre-load of all spring elements is adjustable, preferably independently of each other.

8. The joint according to claim 7, **characterized in that** the respective pre-load can be adjusted in the mounted state of the joint.

9. The joint according to the preamble of claim 1, in particular according to one of the preceding claims, **characterized in that** the second element (4) is connected to a force transmission element (14) of the spring support (8) in such a way that tensile forces and compressive forces can be transmitted.

10. The joint according to one of the preceding claims, **characterized in that** the spring element (10, 12) comprises a helical spring, a helical disc spring, a stack of disc springs and/or a rubber-elastic element, in particular an elastomer block.

11. The joint according to one of the preceding claims, **characterized in that** the at least one spring element (10, 12) applies the first force to the second element (4) only from a first angle of engagement and/or the second force from a second angle of engagement.

12. The joint according to one of the preceding claims, **characterized in that** the damping is adjustable, preferably separately adjustable for both pivot directions.

## Revendications

1. Articulation pour un dispositif orthopédique, l'articulation comprenant
- un premier élément (2),
- un support de ressort (8) ayant au moins un élément élastique (10, 12) et monté sur le premier élément (2), et
- un deuxième élément (4),
dans laquelle le deuxième élément (4) est monté sur le premier élément (2) de manière à pouvoir pivoter dans un premier sens de pivotement à l'encontre d'une première force appliquée par ledit au moins un élément élastique (10, 12) et dans un deuxième sens de pivotement opposé à l'encontre d'une deuxième force appliquée par ledit au moins un élément élastique (10, 12),
le premier élément (2) et le deuxième élément (4) sont disposés l'un sur l'autre de manière à pouvoir pivoter autour d'un axe de pivotement (6),
**caractérisée en ce que** l'articulation comporte une unité d'amortissement hydraulique (46) qui amortit un pivotement du premier élément (2) par rapport au deuxième élément (4) dans au moins un sens de pivotement, de préférence dans les deux sens de pivotement, le support de ressort (8) étant disposé d'un côté de l'articulation et l'unité d'amortissement hydraulique (46) étant disposée de l'autre côté de l'articulation.

2. Articulation selon la revendication 1,
**caractérisée en ce que** le deuxième élément (4) est relié à un élément de transmission de force (14) de l'unité d'amortissement hydraulique (46) de manière à permettre de transmette des forces de traction et des forces de compression.

3. Articulation selon l'une des revendications précédentes,
**caractérisée en ce que** le support de ressort comprend au moins deux éléments élastiques dont l'un applique la première force et l'autre applique la deuxième force.

4. Articulation selon la revendication 3,
**caractérisée en ce que** les deux éléments élastiques (10, 12) sont disposés l'un dans l'autre ou l'un derrière l'autre.

5. Articulation selon la revendication 3 ou 4,
**caractérisée en ce que** les deux éléments élastiques (10, 12) sont des ressorts de compression.

6. Articulation selon la revendication 3 ou 4,
**caractérisée en ce que** lesdits au moins deux éléments élastiques (10, 12) sont disposés et conçus de telle sorte que l'un des deux éléments élastiques (10, 12) est sollicité pour appliquer la force respective dans le sens de la traction et est de préférence un élément élastique de traction, et l'un des deux éléments élastiques est sollicité pour appliquer la force respective dans le sens de la compression et est de préférence un élément élastique de compression.

7. Articulation selon l'une des revendications précédentes,
**caractérisée en ce qu'**une précontrainte de l'un au moins des éléments élastiques (10, 12), de préférence de tous les éléments élastiques, est réglable, de préférence indépendamment les uns des autres.

8. Articulation selon la revendication 7,
**caractérisée en ce que** la précontrainte respective est réglable, à l'état monté de l'articulation.

9. Articulation selon le préambule de la revendication 1, en particulier selon l'une des revendications précédentes,
**caractérisée en ce que** le deuxième élément (4) est relié à un élément de transmission de force (14) du support de ressort (8) de manière à permettre de transmette des forces de traction et des forces de compression.

10. Articulation selon l'une des revendications précédentes,
**caractérisée en ce que** ledit au moins un élément élastique (10, 12) présente un ressort hélicoïdal, un ressort de compression hélicoïdal, une rondelle ressort hélicoïdale, un empilement de rondelles ressorts et/ou un élément présentant l'élasticité du caoutchouc, en particulier un bloc élastomère.

11. Articulation selon l'une des revendications précédentes,
**caractérisée en ce que** ledit au moins un élément élastique (10, 12) n'applique la première force au deuxième élément (4) qu'à partir d'un premier angle d'engagement et/ou n'applique la deuxième force qu'à partir d'un deuxième angle d'engagement.

12. Articulation selon l'une des revendications précédentes,
**caractérisée en ce que** l'amortissement est réglable, de préférence réglable séparément pour les deux sens de pivotement.
